(19) Europäisches Patentamt
European Patent Office
Office européen des brevets

(11) EP 2 724 709 A1

(12) EUROPEAN PATENT APPLICATION

(43) Date of publication:
**30.04.2014 Bulletin 2014/18**

(21) Application number: **12189739.1**

(22) Date of filing: **24.10.2012**

(51) Int Cl.:
***A61K 8/35*** *(2006.01)* ***A61K 8/40*** *(2006.01)*
***A61K 8/44*** *(2006.01)* ***A61Q 17/04*** *(2006.01)*
***A61K 8/86*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Unilever N.V.**
**3013 AL Rotterdam (NL)**

(72) Inventors:
- **Balakrishnan, Lalitha**
  **Whitefield Bangalore (IN)**
- **Raut, Janhavi Sanjay**
  **Beldfordshire MK441LQ (GB)**

(74) Representative: **Acham, Nicholas Clive**
**Unilever NV**
**Patent Group**
**Olivier van Noortlaan 120**
**3133 AT Vlaardingen (NL)**

# (54) A sunscreen composition containing ester salts of L-DOPA

(57) The invention relates to a leave-on sunscreen composition especially to a composition which provides increasing protection of the skin on exposure to uv-radiation. The present inventors have achieved this using a combination of non-ionic surfactant and ester salt of L-DOPA in a sunscreen containing composition.

EP 2 724 709 A1

## Description

### Field of the Invention

The invention relates to a leave-on sunscreen composition especially to a composition which provides increasing protection of the skin on exposure to UV-radiation.

### Background of the invention

Solar radiation includes about 5% ultraviolet (UV) radiation, wavelength of which is between 200 nm and 400 nm. It is further classified into three regions: from 320 to 400 nm (UV-A), 290 to 320 nm (UV-B) and from 200 to 290 nm (UV-C). A large part of UV-C radiation is absorbed by the ozone layer. Scientific studies have indicated that exposure to UV-A and UV-B radiation for short period causes reddening of the skin and localized irritation, whereas continued and prolonged exposure can lead to sunburn, melanoma and formation of wrinkles. It is also reported that UV radiation causes significant damage to hair. Therefore, it is desirable to protect the skin and other keratinous substrates of the human body from the harmful effects of both, UV-A and UV-B radiation.

Various cosmetic preparations have been reported for preventing and/or protecting the skin from harmful effects of ultraviolet radiation. Numerous organic sunscreen agents capable of absorbing UV-A rays are reported in the field of cosmetics amongst which a particularly useful sunscreen is of the dibenzoylmethane class. Many UV-B sunscreens are also known and approved for safe use in personal care compositions for protection from UV-B radiation. Many cosmetic manufacturers prefer to include both UV-A and UV-B sunscreens in photoprotective compositions so as to provide protection over the entire range of UV radiation. Organic sunscreens act by absorbing the UV radiation from the sun and undergo chemical transformation leading to emitting radiation at a different wavelength. Due to the chemical nature of the mode of action, many organic sunscreen are unstable and protection over skin is often achieved only for a few minutes or hours.

This problem is corrected by including actives that stabilize the sunscreens in such compositions. Although sunscreen stablisers are used, they often add to the cost and have the disadvantage that they themselves may interact with some of the other composition ingredients. There is a need in the art to provide for sunscreen actives/ compositions that maintain their efficacy over prolonged time of use. Formulators would be delighted if they can prepare compositions where the sunscreen efficacy is not only maintained but increases with time when such compositions are exposed to UV-radiation, so as to provide consumers with assurance of increased protection over time. A few such sunscreen compositions have been reported and they are known as progressive sunscreens.

Another problem with sunscreens is that in order to provide enhanced sun-protection (high SPF), high amount of sunscreens, e.g. higher than 10, 20 or sometimes 25% sunscreens by weight of the composition are included. Including such high amounts of sunscreens adds to costs and have the disadvantage of interaction with each other and with other composition ingredients thereby lowering their efficacy.

The present inventors have solved both of these problems by developing a sunscreen composition that provides high SPF using relatively low amount of sunscreens and additionally providing progressive sunscreen benefits. The present inventors have achieved this by including a L-dopa ester salt (e.g. L-3,4-dihydroxyphenylalanine methyl ester hydrochloride) in UV-A and UV-B sunscreen containing composition comprising non-ionic surfactant.

US2010/0092410 discloses a liquid skin cleansing composition comprising red petrolatum, a surface treated metal oxide having specific property, at least one sunscreen agent having specific absorption property, a lathering non-ionic surfactant, a lathering anionic surfactant, an alkyl silicone and a volatile cyclic silicone. This patent publication relates to a cleansing composition where the contact time of the composition with the skin is of the order of a few minutes and usually less than a minute. Providing progressive sunscreen benefits where the sun protection factor (SPF) is enhanced over exposure time of an hour or more is not possible with such compositions.

US200440170580 discloses a product comprising at least a UV radiation filtering agent and at least a compound stimulating melanin synthesis.

US4021538 discloses a composition and a method for producing pigmentation in skin or hair by topical application which comprises as active ingredients one or more esters of DOPA. The skin treated with this composition is found to be protected against sun burn and against erythema from ultra-violet light.

The above publications do not teach how to provide high SPF at low sunscreen concentrations.

It is thus an object of the present invention to develop a broad spectrum sunscreen composition which provides high SPF at low sunscreen concentration while exhibiting progressive sunscreen benefits.

### SUMMARY OF THE INVENTION

One aspect of the present invention provides for a high SPF progressive sunscreen leave-on composition

comprising

a) 0.1 to 7% of a UV-A sunscreen;
b) 0.1 to 7% of a UV-B sunscreen;
c) 0.1 % to 10% of a nonionic surfactant; and
d) 0.1 to 10 % of an ester salt of L-DOPA (L-3,4-dihydroxyphenylalanine);

L-DOPA represented by the chemical formula,

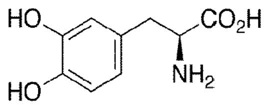

Another aspect of the present invention provides for a method of providing enhanced protection against UV radiation comprising the steps of (a) applying a composition of the invention on to the desired surface and (b) leaving it on for at least 5 minutes.

Yet another aspect of the present invention relates to a kit for providing high SPF progressive sunscreeing effect comprising

(a) a first composition comprising 0.1 to 7% of a UV-A sunscreen, 0.1 to 7% of a UV-B sunscreen and 0.1% to 10% of a nonionic surfactant;

(b) a second composition comprising 0.1 to 10% of an ester salt of L-DOPA (L-3,4-dihydroxyphenylalanine); L-DOPA represented by the chemical formula;

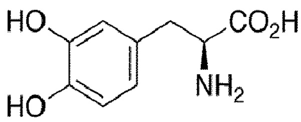

and

(c) instructions for use.

## DETAILED DESCRIPTION OF THE INVENTION

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

"Sunscreen composition" as used herein, is meant to include a composition for topical application to skin and/or hair of mammals, especially humans particularly for sunscreen benefits. Such a composition is to be utilized for application on to the external surface of the body and left on for some time. Such compositions are known as leave-on compositions. The leave-on compositions of the invention are applied to a human body primarily for sun protection but may be used also for other benefits also viz. improving appearance, cleansing, odor control or general aesthetics. The composition of the present invention can be in the form of a liquid, lotion, cream, foam, scrub, gel, or toner, or applied with an implement or via a face mask, pad or patch. Non-limiting examples of personal care compositions include leave-on skin lotions and creams, antiperspirants, deodorants, foundations, or as sunscreen lotions. "Skin" as used herein is meant to include skin on the face and body (e.g., neck, chest, back, arms, underarms, hands, legs, buttocks and scalp) and especially

to the sun exposed parts thereof. The composition of the invention is also of relevance to applications on any other keratinous substrates of the human body other than skin e.g. hair where products may be formulated with specific aim of providing photoprotection.

The present invention is especially useful for providing progressive sunscreening benefits when applied on an external surface of the body. What this means is that when the composition of the invention is applied on the external surface of the body, the efficacy of the composition improves with time of exposure to the sun. The composition comprises a UV-A sunscreen, a UV-B sunscreen, a nonionic surfactant and an ester salt of L-DOPA (L-3,4-dihydroxyphenylalanine).

The composition of the invention comprises a UV-A. A suitable UV-A sunscreen is a dibenzoylmethane compound. A derivative of this compound may also be used although less preferred. Preferred dibenzoylmethane compound is selected from 4-tert-butyl-4'-methoxydibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxydibenzoyl methane, 2,4-dimethyl-4'- methoxy dibenzoylmethane or 2,6-dimethyl-4-tert-butyl-4'-methoxy-dibenzoylmethane. The most preferred dibenzoylmethane derivative is 4-tert.-butyl-4'-methoxydibenzoylmethane. Dibenzoylmethane or its derivative is preferably present in 0.1 to 7%, preferably 0.2 to 5%, more preferably 0.4 to 3%, by weight of the composition.

The composition of the invention comprises a UV-B organic sunscreen. UV-B sunscreen is preferably present in 0.1 to 7%, preferably from 0.5 to 6%, more preferably 1 to 5%, by weight of the composition. The UV-B organic sunscreen is preferably selected from the class of cinnamic acid, salicylic acid, diphenyl acrylic acid, phenylbenzimidazole sulfonic acid or derivatives thereof. A few of the preferred UV-B sunscreens which are commercially available and useful for inclusion in the composition of the invention are Octisalate™ (octyl salicylate), Homosalate™ (3,3,5-trimethyleyclohexyl 2-hydroxybenzoate), Neo Heliopan™ (a range of organic UV filters including ethylhexyl methoxycinnamate (Neo Heliopan AV) and ethylhexyl salicylate (Neo Heliopan OS)), Octocrylene™ (2-ethylhexyl 2-cyano-3,3-diphenyl-2-propenoate) or Parsol MCX™ (2-ethylhexyl-4-methoxycinnamate). According to a particularly preferred aspect of the invention the UVB sunscreen is 2-ethylhexyl-4-methoxycinnamate. According to another particularly preferred aspect of the invention the UVB sunscreen is 2-ethylhexyl 2-cyano-3,3-diphenyl-2-propenoate.

An especially preferred UVB sunscreen is 2-ethylhexyl 2-cyano-3,3-diphenyl-2-propenoate which is commercially available as Octocrylene™. Octocrylene has the chemical formula

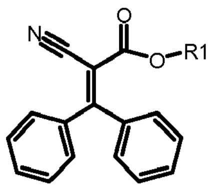

and R1 is a straight or branched chain C1-C30 alkyl group.

A useful aspect of the present invention is that high sun protection values can be obtained even when the total amount of organic sunscreens are present in small amounts which may be in the range of 0.1 to 7%, preferably from 0.5 to 6%, more preferably 1 to 5%, by weight of the composition.

An important ingredient that contributes to the benefits of the present invention is a non-ionic surfactant. The non-ionic surfactant for use in the composition of the present invention has an HLB value (hydrophilic-lipophilic balance) of at least 9. Preferred non-ionic surfactants are selected from the group consisting of ethoxylates of fatty alcohol/fatty acid, alkyl phenol ethoxylates or polyoxyethylene sorbitan alkyl esters.

HLB is calculated using the Griffin method wherein HLB = 20 x Mh / M wherein Mh is the molecular mass of the hydrophilic portion of the molecule and M is the molecular mass of the whole molecule, giving a result on an arbitrary scale of 0 to 20. Typical values for various surfactants are given below:

A value <10 : Lipid soluble (water insoluble)
A value >10 : Water soluble
A value from 4 to 8 indicates an anti-foaming agent
A value from 7 to 11 indicates a W/O (water in oil) emulsifier
A value from 12 to 16 indicates oil in water emulsifier
A value from 11 to 14 indicates a wetting agent
A value from 12 to 15 is typical of detergents
A value of 16 to 20 indicates a solubiliser or hydrotrope

The non-ionic surfactant is preferably selected from the following five classes:

(a) fatty alcohol ethoxylates with saturated carbon chain and having HLB higher than 15.5; or
(b) fatty alcohol ethoxylates with unsaturated carbon chain with HLB higher than 12.
(c) alkyl phenol ethoxylates having HLB higher than 15;
(d) polyoxyethylene sorbitan alkyl esters with saturated C12 to C16 carbon chain and having HLB higher than 12;
(e) polyoxyethylene sorbitan alkyl esters with unsaturated C18 carbon chain and having HLB higher than 9;

Suitable commercially available examples of (a) fatty alcohol ethoxylates with saturated carbon chain and having HLB higher than 15.5 or from (b) the class of fatty alcohol ethoxylates with unsaturated carbon chain with HLB higher than 12 are sold under the brand names Brij 35 (a C12EO23 compound), Brij 97 (unsaturated C18EO12), Brij 700 (C18EO100) or Brij 99 (unsaturated C18EO20). Suitable examples of (c) alkyl phenol ethoxylates with HLB higher than 15 for use in the composition of the invention are sold under the brand names Triton X 165, Triton X 305, Triton 405, or Triton X 705. Suitable examples of (d) polyoxyethylene sorbitan alkyl esters with saturated C12 to C16 carbon chain and having HLB higher than 12 and (e) polyoxyethylene sorbitan alkyl esters with unsaturated C18 carbon chain and having HLB higher than 9 are sold under the brand names Tween20, Tween21, Tween40, Tween80, Tween 81 or Tween85 trioleate. The non-ionic surfactant is included in 0.1 to 10%, preferably 0.1 to 5%, more preferably 0.2 to 4%, further more preferably 0.5 to 3% by weight of the composition.

It is essential as per the present invention to include an ester salt of L-DOPA (L-3,4-dihydroxyphenylalanine); L-DOPA represented by the chemical formula,

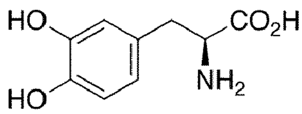

It is particularly preferred that the ester salt of L-DOPA is a methyl ester hydrochloride, an ethyl ester hydrochloride, a propyl ester hydrochloride, a benzyl ester chloride, a cyclohexyl chloride, a phenyl ester hydrochloride, or a butyl ester chloride.

The composition of the invention preferably comprises an antioxidant. Preferred antioxidants are Vitamin C, Vitamin E, sodium sulphite, sodium dithionite or butyl hydroxyl toluene. When present, the antioxidant is present in 0.1 to 1,0%, preferably 0.1 to 0.5% by weight of the composition. Without wishing to be bound by theory, the inventors believe that while antioxidants are not very useful in samples which are freshly prepared, inclusion of antioxidants preserve the progressive sunscreen efficacy on storage of the sample for long time.

The composition of the invention is capable of providing an in-vitro sun protection factor (SPF) of at least 15, preferably 18, more preferably at least 20, further more preferably at least 24.

The individual Sun Protection Factor (SPF) for each subject is defined as the ratio of the amount of energy (dose, in units of $J/m^2$ or in seconds of exposure time) required to produce minimal erythema on protected skin to the amount of energy needed to produce minimal erythema on untreated skin calculated as follows:

$$SPF = \frac{MED \quad \text{Pr}otected \quad Skin}{MED \quad Unprotected \quad Control \quad Site}$$

The SPF for the product is calculated by taking the arithmetic mean of the individual SPF values for all the subjects.

In the laboratory, in vitro SPF is measured using thin film transmittance studies. Thin film transmittance measurements in this case were done using the SPF-290S SPF meter (Optometrics Corporation). The % transmittance of the various compositions was measured using a procedure as outlined below. A roughened PMMA plate was used as the substrate. PMMA is Poly(methyl methacrylate) which is a transparent thermoplastic polymer often used as a lightweight or shatter-resistant alternative to glass. It is transparent to UV light and hence used in these studies. Roughened PMMA is also used to mimic the roughness of human skin. PMMA in the present invention was sourced from Schonberg. 2 $mg/cm^2$ of sample was applied on it, distributed uniformly as small dots using a syringe. Using Para film as a finger cot, the sample was spread on the PMMA Plate uniformly swiping alternately, vertically and horizontally as per the protocol provided by the instrument manufacturers. The sample plate was exposed to a UV lamp and a transmittance scan was done. This scan gives the transmittance as a function of wavelength for a given sample. The reference transmittance scan was obtained using a blank plate, with no sample on the Transpore tape. The transmittance values were used to arrive at the SPF values using the WinSPF software provided with the instrument.

The composition may also comprise an inorganic sunblock. Suitable inorganic sunblocks which may be included

are zinc oxide, titanium dioxide, zinc sulphide, cadmium yellow or Bismuth vanadate. The preferred inorganic sunblocks are titanium dioxide or zinc oxide. The amount of inorganic sunblocks that may be incorporated in the composition is preferably 0.1 to 10%, more preferably 0.5 to 5% by weight of the composition. The inorganic sunblocks preferably have an average particle size in the range of 5 to 100 nm. The inorganic sunblock is preferably hydrophobically coated. Suitable hydrophobic coating materials are aluminium stearate, silicones or ferric stearate.

The composition of the invention may comprises 1 to 25%, preferably 3 to 20%, more preferably 6 to 20% fatty acid by weight of the compostion. In a preferred aspect the composition may include 0.1 to 10% soap. The cosmetically acceptable bases are preferably in a cream, lotion, or emulsion format. A more preferred format is a cream or lotion, further more preferred format is a vanishing cream. Vanishing cream base is one which may comprise 3 to 25%, more preferably 5 to 20% fatty acid. The fatty acids may be saturated or unsaturated fatty acids. The base preferably comprises 0.1 to 10%, more preferably 0.1 to 3% soap. $C_{12}$ to $C_{20}$ fatty acids are especially preferred in vanishing cream bases, further more preferred being $C_{14}$ to $C_{18}$ fatty acids. In creams, the fatty acid is preferably substantially a mixture of stearic acid and palmitic acid. Soaps in the vanishing cream base include alkali metal salt of fatty acids, like sodium or potassium salts The soap is preferably the potassium salt of the fatty acid mixture. It is particularly preferred that the composition comprises at least 6%, preferably at least 10%, more preferably at least 12% fatty acid. The cosmetically acceptable base is usually from 10 to 99.9%, preferably from 50 to 99% by weight of the composition. The cosmetically acceptable base preferably includes water. Water is preferably included in 35 to 90%, more preferably 50 to 85%, further more preferably 50 to 80% by weight of the composition.

The sunscreen composition of the invention is for photo protecting the human epidermis or hair against the damaging effect of solar irradiation, as antisun/sunscreen composition or as makeup product. Such compositions can, in particular, be provided in the form of a lotion, a thickened lotion, a gel, a cream, cleansing milk, an ointment, a powder or a solid tube stick and may optionally be packaged as an aerosol and may be provided in the form of a mousse, foam or a spray.

The personal care compositions of the invention can also contain usual cosmetic adjuvants and skin care additives commonly employed in skin care products such as liquid or solid emollients, silicone oils, emulsifiers, solvents, humectants, polymeric or inorganic thickeners, powders, pigments (example clay mineral, barium sulfate, or pearl pigments, for example silver or gold, or any iris foil pearl pigment, having an interference color of red, orange, green, blue, or, purple (including any iris foil pearl pigments covered with inorganic pigments, organic pigments, laked pigments, etc.), bismuth oxychloride, bismuth oxychloride coated mica,) organic or inorganic sunblocks with and without photostabiliser, skin lightening agents, skin conditioners, optical brighteners, propellants, healing agents (example allantoin), cooling agents (example urea, menthol, menthyl lactate, frescolate), antiseptic agents and other specific skin-benefit actives, skin care actives such as skin lightening actives, antiaging, antiacne, antibacterials, antiperspirant agents etc,. The vehicle may also further include adjuncts such as antioxidants, perfumes, opacifiers, preservatives, colorants and buffers. The necessary amounts of the cosmetic and dermatological adjuvants and additives, based on the desired product, can be chosen by the skilled person.

Vitamins, which act as skin-lightening ingredients can be advantageously included in the composition to provide for additional skin lightening effects. These include vitamin B3, vitamin B6, vitamin C, vitamin A or their precursors and cosmetically acceptable derivatives. Mixtures of the vitamins can also be employed in the composition of the invention. When present, these vitamins are used in the range of 0.01 to 10.0% by weight of said composition.

The invention also relates to a method of providing enhanced protection against UV radiation comprising the steps of (a) applying a composition as claimed in any one of the preceding claims on to the desired surface and (b) leaving it on for at least 5 minutes. Preferably the leave-on composition are left on for at least an hour, more preferably at least 2 hours. The use of such a method is preferably non-therapeutic.

Yet another aspect of the present invention relates to a kit for providing high SPF progressive sunscreeing effect comprising

(i) a first composition comprising 0.1 to 7% of a UV-A sunscreen, 0.1 to 7% of a UV-B sunscreen and 0.1% to 10% of a nonionic surfactant;

(ii) a second composition comprising 0.1 to 10% of an ester salt of L-DOPA (L-3,4-dihydroxyphenylalanine); L-DOPA represented by the chemical formula; instructions for use.

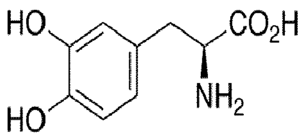

The invention will now be explained in detail with help of the following non-limiting examples, which form

preferred embodiments of the various aspects of the invention.

**EXAMPLES**

Examples A, B and 1: Composition of the invention as compared to those outside the invention.

Compositions as shown in Table- 1 were prepared.

Table - 1

| Ingredient | Example - A, wt% | Example - B, wt% | Example - 1, wt% |
|---|---|---|---|
| Fatty acid@ | 15.0 | 15.0 | 15.0 |
| Avobenzone™ | 0.6 | 0.6 | 0.6 |
| Octocrylene™ | 2.4 | 2.4 | 2.4 |
| Potassium hydroxide | 0.6 | 0.6 | 0.6 |
| Brij -35 | 2.0 | - | 2.0 |
| L-DOPA methyl ester hydrochloride | - | 1.0 | 1.0 |
| Water | To 100 | To 100 | To 100 |

In the Table - 1, Avobenzone™ is chemically 4-t-butyl, 4'-methoxydibenzoylmethane (BMDM or Parsol 1789; was sourced from Chem Spec.

Octocrylene is chemically 2-Ethylhexyl-2-Cyano-3, 3-Diphenyl-2-Propenoate sourced from MERCK

@ Fatty acid used was a mixture of 45% stearic acid and 55% palmitic acid.

Brij -35 is chemically Polyoxyethylene lauryl ether sourced from Croda.

The SPF of samples of Examples A, B and 1 was measured using the following procedure

In the laboratory, in vitro SPF is measured using thin film transmittance studies. Thin film transmittance measurements in this case were done using the SPF-290S SPF meter (Optometrics Corporation). The % transmittance of the various compositions was measured using a procedure as outlined below. A roughened PMMA plate was used as the substrate. 2 $mg/cm^2$ of sample was applied on it, distributed uniformly as small dots using a syringe. Using Para film as a finger cot, the sample was spread on the PMMA Plate uniformly swiping alternately, vertically and horizontally as per the protocol provided by the instrument manufacturers. The sample plate was exposed to a UV lamp and a transmittance scan was done. This scan gives the transmittance as a function of wavelength for a given sample. The reference transmittance scan was obtained using a blank plate, with no sample on the Transpore tape. The transmittance values were used to arrive at the SPF values using the WinSPF software provided with the instrument.

The SPF measured over a time of 0 to 50 minutes is summarised in Table - 2

Table - 2

| Time, minutes | Example - A, SPF | Example - B, SPF | Example - 1, SPF |
|---|---|---|---|
| 0 | 17.2 | 12.0 | 18.7 |
| 10 | 17.2 | 12.7 | 21.1 |
| 20 | 16.8 | 13.0 | 22.3 |
| 30 | 16.7 | 13.1 | 23.4 |
| 40 | 16.4 | 13.2 | 24.3 |
| 50 | 16.1 | 13.4 | 25.1 |

The data in Table - 1 indicates that the composition as per the invention (Example - 1) provides for not only higher SPF values (on an absolute scale) but also progressive sunscreening benefits. Such an advantage is not achieved using compositions outside the invention (Examples A and B).

Examples 2, 3: Effect of inclusion of antioxidants

Compositions as shown in Table - 3 were prepared.

Table - 3

| Ingredient | Example - 2, wt% | Example - 3, wt% |
|---|---|---|
| Fatty acid @ | 15.0 | 15.0 |
| Avobenzone™ | 0.6 | 0.6 |
| Octocrylene™ | 2.4 | 2.4 |
| Potassium hydroxide | 0.6 | 0.6 |
| Brij -35 | 2.0 | 2.0 |
| Methyl ester of L-DOPA | 1.0 | 1.0 |
| Sodium sulphite | - | 0.3 |
| Water | To 100 | To 100 |

The above samples of Example 2 and 3 were stored for 30 days at 25-28 °C and 80-90% RH. The SPF was measured using a procedure similar to Example - 1. The data is shown in Table - 4.

Table - 4

| Time, minutes | Example - 2, SPF | Example - 3, SPF |
|---|---|---|
| 0 | 20.5 | 20.0 |
| 15 | 19.9 | 23.0 |
| 30 | 20.2 | 23.3 |

The data in Table -4 (along with data in Table - 2) indicates that while the sample as per the invention (Example -2), which is stored for one month, demonstrates an enhanced SPF over samples outside the invention (Examples A and B), the progressive effect is not seen. This can be enhanced by inclusion of antioxidants (as seen from Example -3).

The invention thus provides for a high SPF sunscreen composition that demonstrates progressive sunscreen efficacy.

## Claims

**1.** A high SPF progressive sunscreen leave-on composition comprising

a) 0.1 to 7% of a UV-A sunscreen;
b) 0.1 to 7% of a UV-B sunscreen;
c) 0.1 % to 10% of a nonionic surfactant; and
d) 0.1 to 10 % of an ester salt of L-DOPA (L-3,4-dihydroxyphenylalanine); L-DOPA represented by the chemical formula,

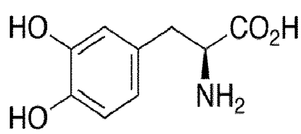

**2.** A composition as claimed in claim 1 wherein said ester salt of L-DOPA is selected from the group consisting of a methyl ester hydrochloride, a ethyl ester hydrochloride, a propyl ester hydrochloride, a benzyl ester chloride, a cyclohexyl chloride, a butyl ester chloride and a phenyl ester hydrochloride.

**3.** A composition as claimed in any one of the preceding claims wherein said non-ionic surfactant is selected from the group consisting of ethoxylates of fatty alcohol/fatty acid, alkyl phenol ethoxylates and polyoxyethylene sorbitan alkyl esters.

**4.** A composition as claimed in any one of the preceding claims wherein said non-ionic surfactant has an HLB value of at least 9.

**5.** A composition as claimed in any one of the preceding claims wherein the UV-B sunscreen is selected from the group consisting of cinnamic acid, salicylic acid, diphenyl acrylic acid, phenylbenzimidazole sulfonic acid and derivatives thereof.

**6.** A composition as claimed in any one of the preceding claims wherein said UVA sunscreen is a dibenzoyl methane compound.

**7.** A composition as claimed in any one of the preceding claims comprising an antioxidant.

**8.** A composition as claimed in claim 7 wherein said antioxidant is selected from the group consisting of Vitamin C, Vitamin E, sodium sulphite, sodium dithionite or butyl hydroxyl toluene.

**9.** A composition as claimed in any one of the preceding claims having an in-vitro SPF of at least 15 preferably 18.

**10.** A method of providing enhanced protection against UV radiation comprising the steps of (a) applying a composition as claimed in any one of the preceding claims on to the desired surface and (b) leaving it on for at least 5 minutes.

**11.** A kit for providing high SPF progressive sunscreeing effect comprising

(a) a first composition comprising 0.1 to 7% of a UV-A sunscreen, 0.1 to 7% of a UV-B sunscreen and 0.1% to 10% of a nonionic surfactant;
(b) a second composition comprising 0.1 to 10% of an ester salt of L-DOPA (L-3,4-dihydroxyphenylalanine); L-DOPA represented by the chemical formula;
(c) instructions for use.

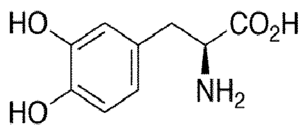

Europäisches Patentamt
European Patent Office
Office européen des brevets

# EUROPEAN SEARCH REPORT

Application Number
EP 12 18 9739

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 983 990 A2 (GIVAUDAN ROURE INT [CH]) 8 March 2000 (2000-03-08)<br>* examples 3,39 *<br>----- | 1,3-7, 9-11 | INV.<br>A61K8/35<br>A61K8/40<br>A61K8/44<br>A61Q17/04<br>A61K8/86 |
| Y | WO 2012/130605 A1 (UNILEVER NV [NL]; UNILEVER PLC [GB]; UNILEVER HINDUSTAN [IN]; CHAVAN M)<br>4 October 2012 (2012-10-04)<br>* page 1, line 4 - line 7 *<br>* page 4, line 26 - page 5, line 7 *<br>* claim 1 *<br>----- | 1-11 | |
| Y,D | US 4 021 538 A (YU RUEY J ET AL)<br>3 May 1977 (1977-05-03)<br>* column 2, line 13 - line 25 *<br>* column 2, line 34 - lines 36,38,50,52 *<br>* column 3, line 32 - line 35 *<br>* column 3, line 36 - line 42 *<br>* column 5, line 18 - line 28 *<br>* example 1 *<br>----- | 1-11 | |
| | | | TECHNICAL FIELDS SEARCHED (IPC) |
| | | | A61K<br>A61Q |

The present search report has been drawn up for all claims

1

| Place of search | Date of completion of the search | Examiner |
|---|---|---|
| Munich | 20 June 2013 | Lenzen, Achim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 12 18 9739

This annex lists the patent family members relating to the patent documents cited in the above-mentioned European search report.
The members are as contained in the European Patent Office EDP file on
The European Patent Office is in no way liable for these particulars which are merely given for the purpose of information.

20-06-2013

| Patent document cited in search report | Publication date | Patent family member(s) | Publication date |
|---|---|---|---|
| EP 0983990 A2 | 08-03-2000 | NONE | |
| WO 2012130605 A1 | 04-10-2012 | NONE | |
| US 4021538 A | 03-05-1977 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20100092410 A
- US 200440170580 A
- US 4021538 A